# EUROPÄISCHE PATENTSCHRIFT

(11) **EP 0 974 841 B1**
(45) Veröffentlichungstag und Bekanntmachung des Hinweises auf die Patenterteilung: **28.05.2008**
(21) Anmeldenummer: 99112023.9
(22) Anmeldetag: 22.06.1999
(51) Int. Cl.: G01N 33/53, G01N 33/574, C07K 14/00

(54) **Entstörung von Immunoassays durch Substanzen, die aus den Framework-Regionen von Antikörpern abgeleitet sind**
Suppression of interference in immunoassays using substances derived from the framework regions of antibodies
Suppression d'interférence dans les immunoessais utilisant les substances dérivés des régions d'encadrement des anticorps

(30) Priorität: 26.06.1998 DE 19828466
(43) Veröffentlichungstag der Anmeldung: 26.01.2000
(73) Patentinhaber: Roche Diagnostics GmbH, 68298 Mannheim (DE)
(72) Erfinder: Nussbaum, Sabine, Dr., 82393 Iffeldorf (DE); Moessner, Ellen, Dr., 74426 Buehlerzell (DE); Lenz, Helmut, Dr., 82327 Tutzing (DE); Praast, Gerald, Dr., 65388 Schlangenbad (DE)

(56) Entgegenhaltungen:
- EP-A- 0 296 544
- US-A- 4 914 040
- KUROKI M ET AL: "Reducing interference from heterophilic antibodies in a two-site immunoassay for carcinoembryonic antigen (CEA) by using a human/mouse chimeric antibody to CEA as the tracer" JOURNAL OF IMMUNOLOGICAL METHODS, ELSEVIER SCIENCE PUBLISHERS B.V.,AMSTERDAM, NL, Bd. 180, Nr. 1, 13. März 1995 (1995-03-13), Seiten 81-91, XP004021073

## Beschreibung

Die vorliegende Erfindung betrifft ein immunologisches Verfahren zum Nachweis eines Analyten in einer Probe, insbesondere von Tumormarkern, das dadurch gekennzeichnet ist, daß zur Entstörung Substanzen, die eine aus den Framework-Regionen der variablen Domäne der Nachweis-Antikörper oder der zur Immuntherapie oder -szintigraphie eingesetzten Antikörper abgeleitete Peptidsequenz enthalten, dem Testansatz zugesetzt werden. Außerdem betrifft die Erfindung die Verwendung solcher Substanzen zur Entstörung von Immunoassays, ein Entstörmittel und ein Verfahren zur Entstörung von Immunoassays durch die genannten Substanzen.

In der Diagnostik haben in den vergangenen Jahren insbesondere immunologische Nachweisverfahren große Bedeutung erlangt. Durch diese Verfahren können Analyten in biologischen Proben nachgewiesen werden. Zu diesen Analyten zählen beispielsweise Arzneimittel, Hormone. Proteine, infektiöse Agenzien. Mikroorganismen und Antikörper gegen diese Analyten. Insbesondere bei der Diagnostik von Krebserkrankungen werden je nach Erkrankung die Tumorantigene oder Tumormarker wie beispielsweise CEA (carcinoembryonales Antigen), PSA (prostataspezifisches Antigen) oder CA125 immunologisch nachgewiesen.

Bei allen immunologischen Nachweisreaktionen kommt es zu einer spezifischen Bindungsreaktion zwischen der Substanz, die nachgewiesen werden soll (Analyt) und mindestens einem spezifischen Bindepartner, der mit dem Analyten spezifisch reagiert oder ihn spezifisch bindet. Der Analyt und der spezifische Bindepartner bilden dabei ein spezifisches Bindungspaar, im allgemeinen ein Komplex zwischen einem Antigen und einem Antikörper oder einem Antikörper-Fragment. Dabei können mehr als ein Analyt oder mehr als ein Bindepartner in jeder Reaktion miteinander reagieren. Die Detektion dieser spezifischen Bindereaktionen kann auf verschiedene Weise erfolgen. Im allgemeinen ist ein Bindepartner der spezifischen Bindereaktion markiert. Übliche Markierungen sind Chromogene, Fluorophore, zur Chemi- oder Elektrochemilumineszenz fähige Substanzen, Radioisotope, Haptene, Enzymmarkierungen oder Substanzen, die wiederum ein spezifisches Bindungspaar bilden können wie beispielsweise Biotin/Streptavidin.

Ein schwerwiegendes Problem bei Immunoassays ist, daß zwischen den spezifischen Bindepartnern des Immunoassays und den Probenbestandteilen unerwünschte Wechselwirkungen und unspezifische Bindereaktionen erfolgen können. Derartige Wechselwirkungen bewirken im allgemeinen eine Erhöhung des Hintergrundsignals, eine stärkere Streuung der Signale und damit eine verringerte Sensitivität und Spezifität des betreffenden Tests. Je nach Art der durch unspezifische Wechselwirkungen hervorgerufenen Störung kann es auch zu falsch-positiven oder zu falsch-negativen Testergebnissen kommen.

Dazu kommt es, wenn in Humanseren Störfaktoren auftreten, die insbesondere mit den häufig als Bindepartner eingesetzten Immunglobulin-Reagenzien eines Immunoassays wechselwirken und diesen dadurch beeinflussen können. Speziell bei Immunoassays, in denen zwei monoklonale Antikörper verwendet werden, kann es zu deutlichen Fehlmessungen mit gegebenenfalls gravierenden Folgen für die weitere Behandlung eines Patienten kommen (Kinders und Hass (1990) Eur.J. Cancer 26(5), 647-648; Boscato und Stuart (1988) Clin Chem. 34(1), 27-33).

Viele dieser Störfaktoren lassen sich unter dem Begriff HAMA (humane Anti-Maus-Antikörper) einordnen. Es handelt sich dabei um Antikörper, die in der zu untersuchenden Probe vorhanden sind und die gegen die spezifischen Antikörper, die als Reagenzien eingesetzt werden, gerichtet sind. Die Bezeichnung HAMA-Störung wird oftmals auch für Störungen durch Antikörper verwendet, die nicht durch Kontakt mit Maus-Immunglobulin entstanden sind oder streng spezifisch für Maus-Immunglobulin sind. Durch diese HAMA-Störung kann es beispielsweise im Falle eines klassischen Sandwichassays trotz Abwesenheit des Analyten zu unspezifischer Vernetzung des an der Festphase gebundenen Antikörpers mit dem markierten Nachweisantikörper kommen. Als Folge davon entsteht ein falsch positives Signal.

Verstärkt wird dieses Problem, da in letzter Zeit in der Diagnostik und Therapie von Tumoren immer häufiger die Immunszintigraphie Anwendung findet. Dabei werden radioaktiv markierte monoklonale Antikörper in die Blutbahn des Patienten injiziert. Die markierten Antikörper binden dann spezifisch an das jeweilige Tumorgewebe. Durch anschließendes Scannen der Radioaktivität beispielsweise mit einer Szintillationskamera kann der Tumor genau lokalisiert werden. Ebenfalls häufig werden immunstimulierende Therapien - auch mit monoklonalen Antikörpern - angewendet, die bewirken sollen, daß im Organismus des Patienten die Bildung von tumorspezifischen Antikörpern zur Bekämpfung des Tumors angeregt wird.

Durch die Anwendung dieser Verfahren treten wie bereits erwähnt vermehrt sehr hohe und zum Teil sehr spezifische HAMA-Titer in Patientenseren auf (siehe beispielsweise Kath et al. (1996) Onkologe 2, 287-296; Holz et al. (1996) Clin. Immunther. 5(3), 214-222; White et al. (1997) Europ. J. of Cancer 33(5), 40; Baum et al. (1993) Hybridoma 12(5), 583-589; Donnerstag et al. (1995) Int. J. Oncology 6, 853-858; Livingston et al. (1995)Vaccine Research 4(2), 87-94; Juweid et al. (1996) Cancer 78(1), 157-168). Diese können auch in Tests, in denen chimäre Antikörper eingesetzt werden, Störungen hervorrufen. Ein wichtiges Beispiel ist der in der Tumortherapie von Colon-Carcinomen (Darmkrebs) in Deutschland zugelassene Antikörper 17-1 A. der die Verkaufsbezeichnung Panorex trägt (Kath et al. (1996), supra; Holz et al. (1996), supra). Darüberhinaus enthält die Liste der in klinischen Studien befindlichen Antikörper zur Therapie eine Vielzahl weiterer Reagentien, die Antikörper enthalten, deren Zulassung immer wahrscheinlicher wird. Bei der Immunszintigraphie sind bereits Antikörper gegen CEA, CA125 und CA72.4 in der klinischen Anwendung, und auch hier ist mit vermehrtem Einsatz zu rechnen.

Im Stand der Technik werden zur Eliminierung dieser Störfaktoren oftmals unspezifische Immunglobuline oder deren Fragmente eingesetzt, die von der gleichen Tierspezies stammen, aus der auch die als Reagenzien im Test eingesetzten Antikörper gewonnen wurden. Den Störfaktoren werden somit quasi alternative Bindungsorte angeboten, an die sie sich anlagern, so daß die spezifische Immunreaktion zwischen Analyt und Antikörpern nicht mehr gestört wird. In der EP-A-0 174 026 wird zur Eliminierung unspezifischer Reaktionen in Immunoassays, in denen als Reagenzien monoklonale Antikörper aus der Maus eingesetzt werden, der Zusatz von Maus- oder Ratten-Serum bzw. -Ascites beschrieben. Eine weitere Möglichkeit zur Vermeidung solcher Störungen ist der Zusatz von aufgereinigten monoklonalen Fab-Fragmenten oder IgG-Molekülen aus der Maus, die gemäß US 4,914,040 besonders in polymerisierter Form eine gute Entstörwirkung entfalten.

Zur Vermeidung falsch-positiver Reaktionen zum Nachweis von CEA wird in der WO 91/16627 die Zugabe einer Mischung von IgG-Molekülen verschiedener Klassen (IgG1, IgG2a, IgG2b) empfohlen. Die Bereitstellung verschiedener Immunglobulinpräparationen in bekannten Mengen und gleichbleibender Qualität erfordert allerdings einen hohen experimentellen Aufwand und betrifft hauptsächlich die konstanten Regionen des Antikörpers.

Die Verringerung des Störpotentials am konstanten Teil des Antikörpers kann auch durch den Einsatz von Fab-Fragmenten anstatt intakter Immunglobuline erfolgen, da die überwiegend vorkommenden Fc-Störungen aufgrund des fehlenden Fc-Teils hier nicht zum Zuge kommen. Die weitere Optimierung stellen humanisierte Antikörper dar, deren Fv-Region aus einem Maus- und einem humanen Anteil zusammengesetzt ist, so daß die Störanfälligkeit auch im variablen Teil noch weiter gesenkt wird (Kuroki et al. (1995) J. of Immunolog. Methods 180, 81-91). Auch die Verwendung von chimären Antikörpern, bei denen der variable Teil aus der Maus und die konstanten Regionen vom Menschen stammen, kann die Störanfälligkeit von diagnostischen Tests senken.

Im Stand der Technik wird zur Vermeidung unspezifisch erhöhter oder erniedrigter Werte beim Nachweis eines Analyten außerdem der Zusatz eines Proteins und insbesondere von Antikörpern, die den als Nachweisreagenzien verwendeten Antikörpern immunologisch verwandt sind, beschrieben (EP-A-0 296 544). Aufgrund der Ähnlichkeit der zugesetzten Antikörper zu den verwendeten Reagenzantikörpern wird die mit diesen interferierende Substanz selektiv abgefangen. Zur Bereitstellung der entstörenden Antikörper wird in der EP-A-0 296 544 empfohlen, die antigenbindenden Strukturen der verwendeten Reagenzantikörper abzuspalten, die antigenbindenden Strukturen durch eine komplementäre Antigenstruktur zu blockieren, die antigenbindende Struktur des verwendeten Reagenzantikörpers durch Mutation zu verändern oder anti-idiotypische Antikörper der gleichen Subklasse einzusetzen.

Mit den Verfahren des Standes der Technik ist es jedoch nicht möglich, die Störungen, die in Proben von zuvor mit immuntherapeutischen Methoden behandelten Patienten beobachtet werden und die je nach eingesetztem Therapie-Antikörper sehr individuell sind, ohne großen experimentellen Aufwand zu beseitigen.

Aufgabe war es daher, ein verbessertes Verfahren zum Nachweis eines Analyten und insbesondere Tumormarkern zu entwickeln, mit dem die Störungen durch unspezifische Substanzen wie HAMA-Störungen weitgehend und besser als mit den Verfahren des Standes der Technik vermieden werden.

Die Aufgabe wird gelöst durch ein neues immunologisches Verfahren zum Nachweis eines Analyten in einer Probe, das dadurch gekennzeichnet ist, daß zur Entstörung Substanzen, die eine aus den Framework-Regionen der variablen Domäne der Nachweis-Antikörper oder der zur Immuntherapie oder -szintigraphie eingesetzten Antikörper abgeleitete Peptidsequenz enthalten, dem Testansatz zugesetzt werden.

Speziell handelt es sich dabei um Substanzen, die Sequenz- und/oder Strukturmerkmale enthalten, die in den Fv-Bereichen von mindestens einem der als Reagenz in einem Immunoassay eingesetzten spezifischen Nachweis-Antikörper vorkommen oder den Sequenzen eines Therapie-/Immunszintigraphie-Antikörpers entsprechen, die die Störungen durch HAMA nahezu vollständig vermeiden. Ein besonders guter Entstöreffekt zeigt sich, wenn die eingesetzten Substanzen Sequenz- bzw. Strukturmerkmale enthalten, die im Framework 1 bzw. Framework 3 der schweren Kette der oder des spezifischen Antikörpers vorkommen.

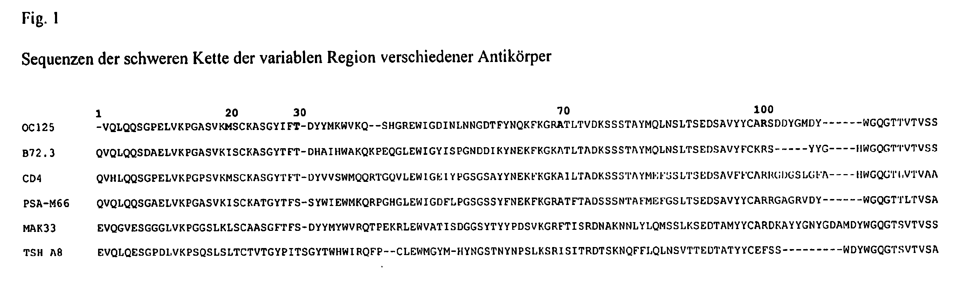

Zur Erläuterung sind in Figur 1 die Sequenzen der schweren Ketten der variablen Region verschiedener Antikörper untereinander dargestellt. Es wird deutlich, daß in der Framework-Region 1 (Aminosäure-Position 1 bis 30) unter den aufgeführten Tumorantikörpem (OC125, B72.3, CD4 und PSA-M66) eine hohe Homologie existiert und zum großen Teil sogar Identität herrscht. Das gleiche gilt für die Framework-Region 3 (Aminosäure-Position 69 bis 100). Auch hier herrscht ein hoher Homologiegrad in den Sequenzen der schweren Ketten der variablen Region der einzelnen Tumorantikörper. Eine vergleichsweise geringe Homologie herrscht dagegen zwischen den Sequenzen der Tumorantikörper und jeweils den Sequenzen der Framework-Regionen 1 und 3 der Nicht-Tumorantikörper MAK33 und einem TSH-Antikörper (TSH = thyroid stimulating hormone). Der Antikörper B72.3 ist beschrieben in Xiang et al. 1990, Mol. Immunol. 27, S. 809-817 sowie in J. Immunology (1993), 151, 6559-6568; der Antikörper MAK33 in Buckel et al. 1987, Gene 51, 13-19; der Antikörper TSH-A8 in der EP-A-0 378 175; der Antikörper OC125 in Tumor Biology (1996). 17, 196-219 und 325-331 sowie in Hybridoma (1994) 13(5), 359-365).

Im Stand der Technik wurde nicht erkannt, daß die immunologische Verwandschaft der Antikörper auf Sequenzhomologien in der variablen Region des Antikörpers zurückzuführen ist. Speziell bei HAMA-Störungen in Patienten-Proben, die aufgrund von Immunszintigraphien oder immunstimulierenden Therapien auch durch tumorantigengerichtete MAKs sehr hohe und spezifische HAMA-Titer aufweisen, ist die erfindungsgemäße Lösung dem Stand der Technik überlegen, da diese Behandlungen mit monoklonalen Antikörpern in den zu untersuchenden Proben zu besonderen, nicht-idiotypischen, aber untereinander ähnlichen HAMA-Störungen insbesondere bei verschiedenen Tumormarkertests führt.

Die Framework-Regionen eines Antikörpers befinden sich im variablen Teil (Fv) des Moleküls, nehmen jedoch im engeren Sinne nicht an der Antigenbindung teil. Sie sind essentiell für die charakteristische Faltung des variablen Teils des Antikörpers und erhalten das Gerüst und die Struktur der Antigenbindungsstelle aufrecht (siehe beispielsweise Roitt et al. (1989) Immunology, Gower Medical Publishing, London, New York, Kapitel 7, Antigen Recognition).

Für das erfindungsgemäße Verfahren werden als Entstörsubstanz bevorzugt Antikörper eingesetzt. Als besonders geeignet hat sich der Einsatz von Antikörpern und deren Fragmenten erwiesen, deren Aminosäure-Sequenz der Framework-Region der Sequenz der als Reagenzien eingesetzten Nachweis-Antikörper bzw. zur Therapie oder Immunszintigraphie verwendeten Antikörper entspricht.

Besonders bevorzugt werden als zur Entstörung verwendete Substanzen die monoklonalen Antikörper B72.3 (J. Immunology (1993), 151, 6559-6568) und OC125 (Tumor Biology (1996), 17, 196-219 und 325-331; Hybridoma (1994) 13(5), 359-365) eingesetzt (siehe auch ISOBM-Bericht: TumorBiology 1996; 17:196-219). Zur Entstörung können auch Antikörper eingesetzt werden, die zu den Antikörpern B72.3 und OC125 äquivalente Strukturmerkmale oder Bindeeigenschaften besitzen.

Wesentlich für die entstörende Wirkung der eingesetzten Antikörper ist die Aminosäure-Sequenz und die Faltungsstruktur im Bereich der Framework-Region 1 oder 3. Sie sollte den in Figur 1 aufgeführten Sequenzen entsprechen oder zumindest eine starke Homologie dazu aufweisen. Die Homologie ist groß genug, wenn sich beim Einsatz des entstörenden Antikörpers noch eine Entstörwirkung feststellen läßt. Prinzipiell sind daher alle Antikörper zur Enstörung geeignet, die eine der in SEQ ID NO 1 bis 8 angegebene Aminosäuresequenz in den Framework-Regionen 1 und/oder 3 besitzen. Die Herkunft und die Erzeugung der zur Enstörung eingesetzten Antikörper ist beliebig. Die monoklonalen Antikörper können aus Ascites-Material stammen oder im Bioreaktor nach dem Fachmann bekannten Methoden hergestellt werden. Die Entstör-Antikörper können, müssen aber nicht aus dem gleichen Organismus stammen wie die Nachweisantikörper.

Die HAMA-Störungen können erfindungsgemäß insbesondere in Verfahren zum Nachweis von Tumormarkern stark verringert werden. Die Entstörung von verschiedenen Tumormarkertests durch das erfindungsgemäße Entstörmittel ist deswegen überraschend, da Tumorantigene in ihren Aminosäuresequenzen und ihrer Struktur durchaus verschieden sind. Dennoch ist es überraschenderweise möglich, verschiedene Tumormarkertests mit einem sequenzhomologen Reagenz zu entstören. Gegenstand der Erfindung ist daher ein immunologisches Verfahren zum Nachweis eines Tumormarkers, das dadurch entstört wird, daß dem Testansatz mindestens eine Substanz - bevorzugt Antikörper - zugesetzt wird, die eine aus den Framework-Regionen der variablen Domäne der Nachweis- oder oder der zur Immuntherapie oder -szintigraphie eingesetzten Antikörper abgeleitete Peptidsequenz enthalten.

Zu den bevorzugt entstörten Tests gehören der Nachweis der Tumormarker CA125, CA15-3, CA19-9, CEA, PSA (in freier und komplexierter Form) und AFP. Erfindungsgemäß werden auch jedoch auch Verfahren zum Nachweis von Analyten, die nicht mit Tumorerkrankungen assoziiert sind, wie beispielsweise Herzkreislauf-Markern (wie Troponin T oder Myoglobin) sehr gut entstört.

Unter dem Begriff Antikörper werden polyklonale und monoklonale Antikörper verstanden. Außerdem werden sowohl der vollständige Antikörper als auch alle bei Immunoassays und sonstigen Verwendungen gängigen Fragmente davon, wie F(ab')₂, Fab' oder Fab-Fragmente, darunter verstanden. Die Herstellung der Antikörper erfolgt nach dem Fachmann geläufigen Verfahren. Umfaßt sind auch solche Antikörper, die durch Veränderung der Antikörper beispielsweise durch gentechnologische Maßnahmen hergestellt wurden. Wesentlich bei allen zur Entstörung eingesetzten Substanzen ist, daß sie eine aus den Framework-Regionen der variablen Domäne Fv der Nachweis-Antikörper abgeleitete Peptidsequenz enthalten.

Unter der Bezeichnung Nachweis-Antikörper werden diejenigen Immunglobuline und ihre Konjugate verstanden, die als Nachweisreagenzien im Test eingesetzt werden. Im Falle eines klassischen Sandwich-Assays gehören zu den Nachweis-Antikörpern der an der Festphase gebundene Antikörper, der den Analyten spezifisch bindet, und der Detektionsantikörper, der einerseits den Analyten spezifisch bindet und andererseits eine Markierung trägt. Sofern zum Nachweis der Markierung weitere Antikörper notwendig sind (beispielsweise bei der Digoxigenin-Markierung, die mit einem enzymmarkierten Anti-Digoxigenin-Antikörper nachgewiesen wird), sind auch diese vom Begriff Nachweis-Antikörper umfaßt.

Erfindungsgemäß können als entstörende Substanzen auch ein oder mehrere Teilstücke der Framework-Regionen - insbesondere der Framework-Regionen 1 und 3 der Fv-Domäne der schweren Immunglobulin-Kette - eingesetzt werden. Bevorzugt sind diese Teilstücke Peptide, die durch enzymatischen Verdau der intakten Antikörper oder auf chemischem Wege nach dem Fachmann bekannten Methoden synthetisiert werden können.

Als Substanzen zur Entstörung von immunologischen Verfahren sind auch Peptide geeignet, die Peptidsequenzen enthalten, die aus den Framework-Regionen 1 und 3 der MAKs OC125, B72.3, PSA oder CD4 stammen.

### Aus der Framework-Region 1:

QVQLQQSGPEASVKMSCKASGYIFT (SEQ ID NO 1 aus MAK OC125)
QVQLQQSDAEASVKISCKASGYTFT (SEQ ID NO 2 aus MAK B72.3)
QVQLQQSGAEASVKISCKATGYTFS (SEQ ID NO 3 aus MAK <PSA>M66)
QVHLQQSGPEPSVKMSCKASGYTFT (SEQ ID NO 4 aus MAK <CD4>)

### Aus der Framework-Region 3:

RATLTVDKSSSTAYMQLNSLT (SEQ ID NO 5 aus MAK OC125)
KATLTADKSSSTAYMQLNSLT (SEQ ID NO 6 aus MAK B72.3)
RATFTADSSSNTAFMEFGSLT (SEQ ID NO 7 aus MAK <PSA>M66)
KAILTADKSSSTAYMEFSSLT (SEQ ID NO 8 aus MAK <CD4>)

Ebenfalls geeignet zur Entstörung sind Teilsequenzen der zuvor genannten Peptide mit einer Länge von mindestens 6 Aminosäuren. Die Peptidsequenzen müssen nicht in isolierter Form vorliegen. Sie können auch von weiteren Aminosäuresequenzen flankiert sein, die nicht aus dem variablen Teil des Antikörpers stammen. Möglich ist auch, daß die Peptide von Kohlenhydrat- oder Lipidresten flankiert sind. Voraussetzung für alle Modifikationen ist jedoch, daß die Entstörwirkung erhalten bleibt und die Modifikationen selbst keine Störungen des Immunoassays bewirken.

Ebenfalls denkbar ist es, daß die zur Entstörung eingesetzten Peptide die entsprechende Sequenz auch mehrfach, also multimer tragen. Sie können also auch als Polyhaptene eingesetzt werden. Dies bedeutet, daß die erfindungsgemäßen Peptide zur Erzeugung eines Polyhaptens, mehrfach auf einen Träger gekoppelt werden. Dabei können auch verschiedene erfindungsgemäße Peptide an einen Träger gekoppelt werden. Als Träger kann ein selbst nicht an der immunologischen Reaktion teilnehmendes Makromolekül, beispielsweise ein größeres Protein wie Rinderserumalbumin, Partikel aus Latex, Polystyrol, Gold oder Dextran dienen. Die Herstellung von Polyhaptenen kann analog zu der in der WO 96/03652 beschriebenen Methode erfolgen.

Vorteilhaft bei den erfindungsgemäßen Substanzen zur Entstörung ist, daß auf eine aufwendige Probenvorbehandlung wie PEG-Fällung, Protein A / G- Chromatographie oder Hitzebehandlung zur Entfernung der HAMA-Interferenzen verzichtet werden kann.

Die Testführungen und Testformate können erfindungsgemäß prinzipiell beliebig gewählt werden. Denkbar sind dem Fachmann geläufige homogene und heterogene Verfahren. Bevorzugt werden heterogene Formate angewendet, bei denen einer der spezifischen Bindepartner direkt oder indirekt an eine Festphase gekoppelt ist. Beispielhaft ist hier der klassische Antigennachweis im Sandwich-Format genannt, der insbesondere bevorzugt bei Tumormarkertests eingesetzt wird. Im Sandwich-Format wird der Analyt (hier ein Antigen) zwischen einem festphasengebundenen und einem markierten Antikörper sandwichartig gebunden. Der Nachweis der Markierung erfolgt nach der Trennung der festen von der flüssigen Phase in einer der Phasen.

Ein weiteres Beispiel für die heterogene Testführung ist der indirekte Nachweis eines Analyt-Antikörpers über dessen Bindung an ein festphasengebundenes Antigen. Der Nachweis erfolgt hier durch die Bindung eines weiteren, markierten Antikörpers an den Analyt-Antikörper.

Ein weiteres Format, das erfindungsgemäß entstört werden kann, ist die kompetitive Testführung, bei der ein festphasengebundener Komplex aus zwei füreinander spezifischen Bindepartnern bzw. Antikörpern gebildet wird, wobei der Bindepartner, der nicht direkt an die Festphase gekoppelt ist, markiert ist. Der Analyt, je nach Testanforderung ein Antigen oder ein Antikörper, verdrängt je nach Konzentration den markierten Bindepartner aus dem Komplex. Nach Trennung der festen von der flüssigen Phase wird die Markierung in einer der Phasen nachgewiesen. Die erfindungsgemäßen entstörenden Substanzen blockieren auch hier die unspezifische Bindung von störenden Probensubstanzen an die als Nachweisreagenzien eingesetzten Bindepartner/Antikörper und verhindern falsche Testergebnisse weitgehend.

Die Konzentrationen im Probenansatz, in denen die zur Entstörung eingesetzten Antikörper verwendet werden, sind nach oben nur durch die Löslichkeit der Antikörper in wässrigem Medium limitiert. Als geeignet haben sich Mindestkonzentrationen von etwa 10 µg/ml bis 40 µg/ml und als sinnvolle Obergrenze ca. 2 mg/ml, bevorzugt 250 µg/ml Antikörper erwiesen.

Es können neben den erfindungsgemäßen Substanzen auch weitere entstörende Maßnahmen ergriffen werden, sofern dies erforderlich ist. Dazu zählen beispielsweise der Zusatz von MAK33 (Boehringer Mannheim GmbH, Deutschland, Ident-Nr. 1200 941) oder gleichartig wirkenden Antikörpern, polyMAK33 (polymeres Maus-IgG, Boehringer Mannheim GmbH, Deutschland, Ident-Nr. 1368 338), RSA, Salzen, Detergenzien.

Neben den sogenannten Naßtesten, bei denen die Testreagenzien in flüssiger Phase vorliegen, können auch alle gängigen Trockentestformate, die zum immunologischen Nachweis von Analyten geeignet sind, verwendet werden. Bei diesen Trockentests oder Teststreifen, wie sie beispielsweise in der EP-A-0 186 799 beschrieben sind, sind die Testkomponenten auf einem Träger aufgebracht. Das erfindungsgemäße Entstörmittel ist dann bereits vor der immunologischen Reaktion auf dem trockenen Teststreifen aufgebracht.

Das erfindungsgemäße Entstörmittel sollte bei Naßtesten bevorzugt vor oder gleichzeitig mit der Zugabe der als Nachweisreagenzien eingesetzten Bindepartner zur Probe gegeben werden, um eine möglichst frühe Reaktion der störenden, unspezifischen Substanzen mit dem Entstörmittel zu ermöglichen.

Als Proben für die Durchführung von den erfindungsgemäß zu entstörenden Immunoassays können alle dem Fachmann geläufigen biologischen Flüssigkeiten verwendet werden. Bevorzugt werden als Probe Körperflüssigkeiten wie Vollblut, Blutserum, Blutplasma, Urin oder Speichel eingesetzt.

Ein weiterer Gegenstand der Erfindung ist ein Entstörmittel, enthaltend mindestens eine Substanz, die eine aus den Framework-Regionen der variablen Domäne der Nachweis-Antikörper oder der zur Immuntherapie oder -szintigraphie eingesetzten Antikörper abgeleitete Peptidsequenz enthält. Als weitere Bestandteile des Entstörmittels sind dem Fachmann geläufige Puffer, Salze und Detergenzien möglich. Das Entstörreagenz kann in flüssiger, wässriger Form oder in lyophilisierter Form bereitgestellt werden.

Ebenfalls ein Gegenstand der Erfindung ist ein Verfahren zur Entstörung von Immunoassays, das dadurch gekennzeichnet ist, daß zur Entstörung insbesondere von HAMA-Effekten Substanzen, die eine aus den Framework-Regionen der variablen Domäne der Nachweis-Antikörper oder der zur Immuntherapie oder -szintigraphie eingesetzten Antikörper abgeleitete Peptidsequenz enthalten, dem Testansatz zugesetzt werden.

Die folgenden Beispiele erläutern die Erfindung weiter.

### Beispiel 1

### Entstörung des CA125-Tests mit verschiedenen Entstörreagenzien

Die Durchführung des CA125-Nachweises erfolgt entsprechend der in der Packungsbeilage zum Enzymun-Test^{®} CA 125 II der Boehringer Mannheim GmbH Deutschland (Ident. Nr. 1289 004) angegebenen Vorgehensweise. Testprinzip ist ein 1-Schritt-Sandwich-ELISA beruhend auf der Streptavidin-Technologie. Es werden zwei monoklonale CA125-spezifische Antikörper eingesetzt, die unterschiedliche Epitope auf dem CA125 spezifisch erkennen. Einer der Antikörper ist mit Biotin, der andere mit Peroxidase markiert. Die Probe wird zusammen mit einer Inkubationslösung, die biotinylierte Anti-CA125-Antikörper und mit Peroxidase (POD) markierte Anti-CA125-Antikörper enthält, in ein mit Streptavidin beschichtetes Kunststoffröhrchen gefüllt. Während der Inkubation bindet der biotinylierte Antikörper an die Festphase. Das in der Probe vorhandene CA125 bindet an den biotinylierten Antikörper. Der mit POD markierte Antikörper bindet wiederum an das vom biotinylierten Antikörper gebundene CA125.

Die Detektion der an die Festphase gebundenen Sandwich-Komplexe erfolgt nach einem Wasch-Schritt über eine Indikatorreaktion. Dazu wird eine Substrat-Chromogen-Lösung, die ABTS^{®} (2,2'-Azino-di-[3-ethyl-benzthiazolin-sulfonsäure (6)]-diammoniumsalz) und H₂O₂ enthält, zum Ansatz gegeben. Durch die enzymatische Aktivität der Peroxidase entwickelt sich in Abhängigkeit der Menge des gebundenen PODmarkierten Antikörpers ein Farbstoff, der photometrisch nachgewiesen wird.

Zu den Testansätzen werden gleichzeitig mit Zugabe des biotinylierten Antikörpers 40 µg/ml verschiedene Entstörreagenzien des Standes der Technik sowie zum Vergleich das erfindungsgemäße Entstörreagenz dazugegeben.

Als Entstörreagenzien werden die erfindungsgemäße Lösung anhand des Beispieles MAK B72.3 (intaktes IgG), ein HAMA-Entstörreagenz der Firma Trina (Maus-IgG), ein HAMA-Entstörreagenz der Firma Scantibodies (HBR, monoklonales Anti-human-IgM), Entstörreagenz MAK33 der Boehringer Mannheim GmbH Deutschland (Ident-Nr.: 1 200 941), Entstörreagenz pMAK33 der Boehringer Mannheim GmbH Deutschland (polymerisiert. Ident-Nr. 1 096 478) sowie drei Tumormarkerantikörper gegen PSA. AFP und CEA eingesetzt.

Als Proben werden Serum-Proben von Patientinnen mit Ovarkarzinom und erfolgter Immunszintigraphie sowie käufliche HAMA-Panels sowie ein Normalserum (NS) eines gesunden Patienten eingesetzt. Tabelle 1 zeigt die Meßwerte des CA125-Tests unter Zusatz verschiedener Entstörreagenzien. Eine Unit POD (1U) entspricht der enzymatischen Aktivität, die 1 µmol ABTS in einer Minute bei 25°C und pH 5 oxidiert.

**Tabelle 1: Meßwerte mit dem CA125-Test**

| | **CA125 ohne Zusatz U/ml** | **CA125 + B72.3 U/ml** | **CA125 + Trina.13 U/ml** | **CA125 + HBR U/ml** | **CA125 + MAK33 U/ml** | **CA125 + pMAK3 U/ml** | **CA115 + <PSA>M66 U/ml** | **CA 125 + <AFP>MTull U/ml** | **CA125 + <CEA>MTu3 U/ml** |
|---|---|---|---|---|---|---|---|---|---|
| **Normalserum** | **16.42** | 23.4 | 20.64 | 15.48 | 14.62 | 15.12 | 9.25 | 12.5 | 7.16 |
| **Immunszintigraphie mit B43.13** | **>496** | 107.4 | 124.68 | 143.05 | 434.29 | 433.36 | 221,04 | 350.42 | 369.78 |
| **(Ovarkarzinom)** | | | | | | | | | |
| **Immunszintigraphie mit B43.13** | **129** | 118.6 | 117.54 | 117.13 | 134.43 | 126.6 | 125.8 | 141.98 | 130.1 |
| **(Ovarkarzinom)** | | | | | | | | | |
| **Immunszintigraphie mit B43.13** | **262** | 96.5 | 106.65 | 117.31 | 158.04 | 160.89 | 111.9 | 157.97 | 158.63 |
| **(Ovarkarzinom)** | | | | | | | | | |
| **Käufliches HAMA-Panel III** | **111.7** | - | 34.42 | 35.3 | 50.77 | 55.65 | - | - | - |
| **(Bioclinical Partners)** | | | | | | | | | |
| **Immunszintigraphie mit OC125** | **>496** | 289.7 | 329.93 | 322.77 | 348.52 | 360.41 | 300.2 | 402.03 | 335.33 |
| **(Ovarkarzinom)** | | | | | | | | | |
| **Käufliches HAMA-Panel** | **382.2** | 22.2 | 115.05 | 137.75 | 95.6 | 111.84 | 114.6 | 129.03 | 120.02 |
| **(Starrate D)** | | | | | | | | | |
| **Immunszintigraphie mit B43.13** | **>496** | 158 | 404.09 | 420.97 | >496 | >496 | - | - | - |
| **(Ovarkarzinom)** | | | | | | | | | |

Der CA125-Test zeigt ohne Entstörreagenz positive Meßwerte, wobei es sich hier um falsch positive Werte handelt. Es kann gezeigt werden, daß mit Hilfe des monoklonalen Antikörpers B72.3 die beste HAMA-Entstörung, d.h. der niedrigste Meßwert erzielt wird. Damit ist das erfindungsgemäße Entstörmittel denen des Standes der Technik klar überlegen. Von den Tumormarker-Antikörpern erweist sich der PSA-Antikörper als der zur Entstörung am besten geeignete, was in Analogie zur Sequenzhomologie steht.

### Beispiel 2:

### Entstörung des CA125-Tests mit <CD4>M3-10, MAK33 und dem Panorex-Antikörper

Der Nachweis für CA125 wird entsprechend Beispiel 1 durchgeführt. Als Entstörreagenzien werden im Vergleich das erfindungsgemäße Entstörreagenz (hier: MAK B72.3), polymerisierter und nicht polymerisierter MAK33 wie in Beispiel 1, der Antikörper <CD4>M3-10 sowie der Panorex-Antikörper eingesetzt. Die Zugabe der Entstörreagenzien erfolgt mit einer Konzentration von 40 µg/ml.

**Tabelle 2: Meßwerte mit dem CA125-Test**

| | **CA125 ohne Zusatz U/ml** | **CA125 + B72.3 U/ml** | **WDF %** | **CA125 + Panorex U/ml** | **WDF %** | **CA125 + <CD4> U/ml** | **WDF %** | **CA125 + PMAK33 U/ml** | **WDF %** | **CA125 + MAK33 U/ml** | **WDF %** |
|---|---|---|---|---|---|---|---|---|---|---|---|
| **TMC1** | 43.05 | 42.88 | **99** | 44.73 | **104** | 38.98 | **90** | 47.97 | **111** | 40.41 | **94** |
| **TMC2** | 88.68 | 90.13 | **102** | 94.37 | **106** | 85.45 | **96** | 92.77 | **105** | 87.53 | **99** |
| **Normalserum** | 21.13 | 19.43 | **92** | 21.99 | **104** | 15.22 | **72** | 23.19 | **110** | 21.89 | **104** |
| **Käufliches HAMA-Panel 1** | 60.83 | 39.47 | **65** | 50.8 | **84** | 40.41 | **66** | 48.3 | **80** | 42.58 | **70** |
| **(Bioclinical Partners)** | | | | | | | | | | | |
| **Immunszintigraphie mit B43.13** | 142.94 | 77.44 | **54** | 93.98 | **66** | 77.61 | **54** | 89.87 | **63** | 77.82 | **54** |
| **(Ovarkarzinom)** | | | | | | | | | | | |
| **Käufliches HAMA-Panel** | 408.08 | 38.62 | **9** | 57.06 | **14** | 42.63 | **10** | 57.16 | **14** | 67.83 | **17** |
| **(StarrateD)** | | | | | | | | | | | |
| **Käufliches HAMA Panel II** | >519 | 69.2 | **<13** | 513.82 | **<99** | 77.78 | **<15** | 111.72 | **<21** | 147.35 | **28** |
| **(Bioclinical Partners)** | | | | | | | | | | | |
| **Immunszintigraphie mit OC125** | >519 | 358.66 | **<70** | 1350.1 | - | 465.88 | **90** | >519 | - | 1189.08 | - |
| **(Ovarkarzinom)** | | | | | | | | | | | |
| **Immunszintigraphie mit B43.13** | 239.26 | 39.64 | **17** | 119.11 | **50** | 45.84 | **19** | 103.22 | **43** | 112.67 | **47** |
| **(Ovarkarzinom)** | | | | | | | | | | | |
| **Immunszintigraphie mit B43.13** | 211.88 | 43.73 | **21** | 71.92 | **34** | 62.07 | **29** | 83.91 | **40** | 108.34 | **51** |
| **(Ovarkarzinom)** | | | | | | | | | | | |

| | | | | | | | | | | | |
|---|---|---|---|---|---|---|---|---|---|---|---|
| % WDF: prozentuale Wiederfindung, erhaltene Menge bezogen auf den Test ohne Zusatz von Entstörreagenz TMC1 und TMC2: Tumormarkerkontrolle der Boehringer Mannheim GmbH. Deutschland (Ident.-Nr. 1 489 666), Kontrollserum auf Humanserumbasis mit 2 Sollwerten, enthält u.a. AFP, CEA. PSA, frees PSA, CA125 | | | | | | | | | | | |

Es zeigt sich, daß mit dem Sequenzhomologen <CD4>-Antikörper eine mit dem Antikörper B72.3 vergleichbare Entstörung erzielt wird, daß aber der weniger sequenzhomologe Panorex-Antikörper eine sehr viel schlechtere Entstörwirkung (nur eine geringe Erniedrigung der Signale) zeigt. Darüberhinaus wird die deutlich schlechtere Wirkung der Entstörreagenzien des Standes der Technik deutlich.

### Beispiel 3

### Entstörung des CEA-Tests mit verschiedenen Entstörreagenzien

Um neben dem CA125-Test ein zweites Kriterium für die Entstörwirkung zu haben, wurde der CEA-Enzymun^{®}-Test der Boehringer Mannheim GmbH (Ident.-Nr. 1448021) als Matrix gewählt. Die Durchführung erfolgt entsprechend der in der Packungsbeilage beschreibenen Vorgehensweise. Es handelt sich in Analogie zum CA125-Test hier ebenfalls um einen 1-Schritt-Sandwich-ELISA, der auf der Biotin-Streptavidin-Technologie beruht. Es werden zwei monoklonale Antikörper CEA-spezifische Antikörper eingeseetzt, die unterschiedliche Epitope auf dem CEA erkennen. Einer der Antikörper ist mit Biotin, der andere mit Peroxidase markiert. Die Probe wird zusammen mit einer Inkubationslösung, die biotinylierte Anti-CEA-Antikörper und mit Peroxidase (POD) markierte Anti-CEA-Antikörper enthält, in ein mit Streptavidin beschichtetes Kunststoffröhrchen gefüllt. Während der Inkubation bindet der biotinylierte Antikörper an die Festphase. Das in der Probe vorhandene CEA bindet an den biotinylierten Antikörper. Der mit POD markierte Antikörper bindet wiederum an das vom biotinylierten Antikörper gebundene CEA.

Die Detektion der an die Festphase gebundenen Sandwich-Komplexe erfolgt wie in Beispiel 1 und 2 über die dort beschriebene Indikatorreaktion.

Als Entstörreagenzien werden solche aus den Beispielen 1 und 2 gewählt, sowie der OC125-Antikörper eingesetzt. Die Zugabe erfolgt mit einer Konzentration von 10 µg/ml.

**Tabelle 3: Meßwerte für den CEA-Test**

| | **CEA Enzymun ng/ml** | **CEA Enzymun + OC135 ng/ml** | **CEA Enzymun + B72-3 ng/ml** | **CEA Enzymun + HBR ng/ml** | **CEA Enzymun + MAK33 ng/ml** |
|---|---|---|---|---|---|
| **Normalserum** | **0,79** | **1,01** | **-** | **1** | **1,1** |
| **Immunszintigraphie mit B43.13** | **6,8** | **2,25** | **2,78** | **2,77** | **5,3** |
| **(Ovarkarzinom)** | | | | | |
| **Immunszintigraphie mit B43.13** | **7,84** | **3,01** | **3,62** | **3,4** | **6,09** |
| **(Ovarkarzinom)** | | | | | |
| **Käufliches HAMA-Panel III** | **>51,6** | **4,98** | **6,89** | **9,1** | **17,72** |
| **(Bioclinical Partners)** | | | | | |
| **Immunszintigraphie mit B43.13** | **13,7** | **3,9** | - | **5,32** | **8,14** |
| **(Ovarkarzinom)** | | | | | |
| **Käufliches HAMA-Panel** | **5,77** | **2,94** | **2,8** | **2,1** | **3,95** |
| **(Starrate D)** | | | | | |
| **Käufliches HAMA-Panel II** | **12,97** | **4,2** | **3,62** | **4,42** | **5,36** |
| **(Bioclinical Partners)** | | | | | |
| **Immunszintigraphie mit <CEA>** | **48,62** | **10,45** | **8,28** | **8,67** | **19,8** |
| **(Colonkarzinom)** | | | | | |

Die Ergebnisse mit den erfindungsgemäßen Entstörreagenzien MAK B72.3 und OC125 sind in etwa vergleichbar. Diese beiden Antikörper sind bis auf wenige Aminosäuren sequenzhomolog in den Framework-Regionen 1 und 3. Der Stand der Technik liefert deutlich schlechtere Ergebnisse bei der Entstörung.

### Beispiel 4

### Entstörung verschiedener Testsysteme

Im Vergleich werden verschiedene Tumormarker-Tests gezeigt, die einerseits auf der Enzymun^{®}-Testführung beruhen (s. Beispiele 1 bis 3) und andererseits nach dem Elecsys^{®} Verfahren der Boehringer Mannheim GmbH durchgeführt werden. Die Elecsys^{®}- Testführung für alle Nachweise beruht auf dem 1-Schritt-Sandwich-Prinzip mit zwei Antikörpern, die verschiedene Epitope auf den Analyten (Tumormarkern) erkennen, analog zu Beispiel 3. Die Festphase wird von magnetischen, mit Streptavidin beschichteten Latexbeads gebildet, an die ein biotinylierter, für den Tumormarker spezifischer Antikörper bindet. Die Detektion des gebundenen Tumormarkers erfolgt nach der Trennung der festen von der flüssigen Phase durch die Messung der Elektrochemilumineszenz, die von einem zweiten Tumormarker-spezifischen, mit einem Rutheniumkomplex markierten Antikörper ausgeht.

Hier wurde die Entstörwirkung der erfindungsgemäßen Lösung an verschiedenen Enzymun^{®} und Elecsys^{®}-Tests untersucht:
CEA-Enzymun^{®} (Ident.-Nr. 1448021; CEA Elecsys^{®} (Ident.-Nr. 1731629); CA125 Enzymun^{®} (Ident.-Nr. 1289004), CA125 Elecsys^{®} (Ident.-Nr. 1776223); AFP Elecsys^{®} (Ident.-Nr. 1731327); PSA tot. Enzymun^{®} (Ident.-Nr. 1555332); PSA tot. Elecsys^{®} (Ident.-Nr. 1731262); PSA free Enzymun^{®} (Ident.-Nr. 1776444); PSA free Elecsys^{®} (Ident.-Nr. 1820800).

Das Entstörreagenz MAK B72.3 wird in einer Konzentration von 40 µg/ml zugesetzt.

Bei den Proben handelt es sich um hochtitrige HAMA-Seren aus Immuntherapie oder - szintigraphie.

**Tabelle 4: Entstörung verschiedener Testsystem**

| | **CA125 Enz.** | **CA125 Enz. + B72.3** | **CA125 ECL** | **CA125 ECL + B72.3** | **AFP ECL** | **AFP ECL + B72.3** | **PSA total Enz.** | **PSA total Enz. + B72.3** | **PSA tot. ECL** | **PSA tot. ECL + B72.3** | **PSA frei Enz.** | **PSA frei Enz. + B72.3** | **PSA frei ECL** | **PSA frei ECL + B72.3** |
|---|---|---|---|---|---|---|---|---|---|---|---|---|---|---|
| **Serum 1** | **339,1** | 24.9 | 21.6 | - | **16,98** | 12 | **1,62** | 0.2 | 0.73 | 0.2 | **0,35** | 0.12 | 0.73 | 0.15 |
| **Serum 2** | **>496** | 52.7 | 72.71 | 46.6 | **6,36** | 2.81 | **0,48** | 0 | 1.21 | 0.36 | **1,04** | 0.28 | 2.6 | 0.21 |
| **Serum 3** | **>496** | 370.2 | 186.6 | 133.1 | **4,73** | 1.87 | **0,75** | 0 | 1.12 | 0.17 | **1,62** | 0.32 | 4.93 | 0.27 |
| **Serum 4** | **>496** | 82.3 | 59.6 | 30.1 | **16,07** | 5.9 | **1,3** | 0.13 | 1.7 | 0.52 | **2,84** | 0.39 | 13.1 | 0.44 |
| **Serum 5** | **205,8** | 23.01 | 24.3 | - | **2,33** | 1.85 | **0,07** | 0 | 0.27 | 0.05 | **0,11** | 0.03 | 2.23 | 0.12 |
| **Serum 6** | **181,1** | 28.65 | 29.62 | - | **2,03** | 1.52 | **0,07** | 0 | 0.38 | 0.1 | **0,25** | 0.08 | 3.57 | 0.24 |
| **Serum 7** | - | - | - | - | **2** | 1.24 | **0,14** | 0 | 0.41 | 0.14 | **0,45** | 0.18 | 2.7 | 0.34 |

| | | | | | | | | | | | | | | |
|---|---|---|---|---|---|---|---|---|---|---|---|---|---|---|
| Enz. = Enzymun^{®} ECL = Elecsys^{®} | | | | | | | | | | | | | | |

Bei CA125 zeigen beide Tests eine deutliche Verringerung der HAMA-Störung durch Zugabe von B72.3.

Ebenso wird das Störpotential bei AFP, PSA gesamt und frei gesenkt. Für PSA frei gilt allerdings, daß die Störanfälligkeit des Elecsys® -Tests über dem Enzymun-Test liegt. Es kann also eine Entstörung unabhängig vom Testsystem gezeigt werden.

### Beispiel 5:

### Entstörung des Troponin T- und Myoglobin-Tests

Um die Entstörwirkung auch an anderen Parametern als den Tumormarkern zu zeigen, werden die Herzkreislauf-Marker TroponinT (Ident.-Nr.: 1 731 351) und Myoglobin (Ident.-Nr.: 1 820 788) gewählt.

Das Testprinzip beruht auf dem in Beispiel 4 erläuterten Elecsys^{®}-Test. Es werden zwei monoklonale Antikörper gegen Troponin T beziehungsweise Myoglobin eingesetzt, die verschiedene Epitope auf dem Analyten (TroponinT bzw. Myoglobin) erkennen. Einer der MAKs ist mit Biotin, der andere mit einem Rutheniumkomplex markiert, dessen Elektrochemilumineszenz-Signal gemessen wird.

Es werden 100 µg/ml Entstörreagenz MAK B72.3 zu den Testansätzen dazugegeben.

**Tabelle 5: Entstörung von Tests zum Nachweis von Troponin T und Myoglobin**

| | **Myoglobin** | **Myoglobin + 100µg/ml B72.3** | | **Troponin T** | **Troponin T + 100µg/ml B72.3** | |
|---|---|---|---|---|---|---|
| | **(ng/ml)** | **(ng/ml)** | **WDF%** | **(ng/ml)** | **(ng/ml)** | **WDF %** |
| **Kontrolle 1** | 99.229 | 100.396 | **101** | 0.148 | 0.148 | **100** |
| **Kontrolle 2** | 1312.549 | 1283.688 | **98** | 7.261 | 7325 | **101** |
| **Immuntherapie mit B43.13 (Ovarkarzinom)** | 19.819 | 14.888 | **75** | 0.015 | 0.011 | **73** |
| **Käufliches HA.MA-Panel (Starrate D)** | 334.348 | 60.72 | **18** | 0.119 | 0.073 | **61** |
| **Käufliches HAMA-Panel (Bioclinical Partners)** | 210.342 | 42.318 | **20** | 0.187 | 0.121 | **65** |
| **Immuntherapie mit OC125 (Ovarkarzinom)** | 231.522 | 46.914 | **20** | 0.178 | 0.106 | **60** |
| **Immunszintigraphie - <CEA>- MAKs** | 56.877 | 21.099 | **37** | 0.056 | 0.026 | **46** |
| **Immuntherapie mit B43.13 (Ovarkarzinom)** | 31.062 | 19.405 | **62** | 0.061 | 0.031 | **51** |

Auch hier bringt der Zusatz von B72.3 deutlich niedrigere Signale und hebt die falsch positive Messung auf. Die Anwendung der erfindungsgemäßen Entstörreagenzien ist damit auch über die Tumormarker hinaus gezeigt.

## Patentansprüche

1. Immunologisches Verfahren zum Nachweis eines Analyten in einer Probe **dadurch gekennzeichnet, daß** zur Entstörung Substanzen, die eine Peptidsequenz gemäß SEQ ID NO 1- 8 oder Teilsequenzen davon mit einer Länge von mindestens 6 Aminosäuren enthalten, dem Testansatz zugesetzt werden.

2. Verfahren nach Anspruch 1, **dadurch gekennzeichnet, daß** es sich um einen Tumormarkertest handelt.

3. Verfahren nach Anspruch 1 oder 2, **dadurch gekennzeichnet, daß** die zur Entstörung eingesetzten Substanzen Antikörper oder deren Fragmente sind.

4. Verfahren nach Anspruch 1 oder 2, **dadurch gekennzeichnet, daß** die zur Entstörung eingesetzten Substanzen Peptide sind.

5. Verwendung von Substanzen, die eine Peptidsequenz gemäß SEQ ID NO 1-8 oder Teilsequenzen davon mit einer Länge von mindestens 6 Aminosäuren enthalten, zur Entstörung von Immunoassays.

6. Verfahren zur Entstörung von Immunoassays, **dadurch gekennzeichnet, daß** zur Entstörung Substanzen, die eine Peptidsequenz gemäß SEQ ID NO 1-8 oder Teilsequenzen davon mit einer Länge von mindestens 6 Aminosäuren enthalten, dem Testansatz zugesetzt werden.

## Claims

1. Immunological method for the detection of an analyte in a sample, **characterized in that** substances which contain a peptide sequence according to SEQ ID NO 1-8 or partial sequences thereof having a length of at least 6 amino acids are added to the test mixture to reduce interference.

2. Method according to claim 1, **characterized in that** it is a tumour marker test.

3. Method according to claim 1 or 2, **characterized in that** the substances used to reduce interference are antibodies or fragments thereof.

4. Method according to claim 1 or 2, **characterized in that** the substances used to reduce interference are peptides.

5. Use of substances which contain a peptide sequence according to SEQ ID NO 1-8 or partial sequences thereof having a length of at least 6 amino acids, to reduce interference in immunoassays.

6. Method for reducing interference in immunoassays, **characterized in that** substances which contain a peptide sequence according to SEQ ID NO 1-8 or partial sequences thereof having a length of at least 6 amino acids are added to the test mixture to reduce interference.

## Revendications

1. Procédé immunologique pour le décèlement d'un analyte dans un échantillon, **caractérisé en ce que**, pour le déparasitage, on ajoute, à la préparation d'essai, des substances qui contiennent une séquence peptidique conformément aux SEQ ID NO 1 - 8 ou des séquences partielles de ces dernières, possédant une longueur d'au moins 6 acides aminés.

2. Procédé selon la revendication 1, **caractérisé en ce qu'**il s'agit d'un test visant des marqueurs tumoraux.

3. Procédé selon la revendication 1 ou 2, **caractérisé en ce que** les substances mises en oeuvre pour le déparasitage sont des anticorps ou leurs fragments.

4. Procédé selon la revendication 1 ou 2, **caractérisé en ce que** les substances mises en oeuvre pour le déparasitage sont des peptides.

5. Utilisation de substances qui contiennent une séquence peptidique conformément aux SEQ ID NO 1 - 8 ou des séquences partielles de ces dernières, possédant une longueur d'au moins 6 acides aminés pour le déparasitage d'immunodosages.

6. Procédé pour le déparasitage d'immunodosage, **caractérisé en ce que**, pour le déparasitage, on ajoute, à la préparation d'essai, des substances qui contiennent une séquence peptidique conformément aux SEQ ID NO 1 - 8 ou des séquences partielles de ces dernières, possédant une longueur d'au moins 6 acides aminés.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Str. 116
      (C) ORT: Mannheim
      (E) LAND: DE
      (F) POSTLEITZAHL: 68305
      (G) TELEFON: 06217593215
      (H) TELEFAX: 06217594457
   (ii) BEZEICHNUNG DER ERFINDUNG: Entstoerung von Immunoassays durch Substanzen, die aus den Framework-Regionen von Antikoerpern abgeleitet sind
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER JETZIGEN ANMELDUNG: ANMELDENUMMER: DE 19828466.7
(2) ANGABEN ZU SEQ ID NO: 1:
   (i) SEQUENZKENNZEICHEN:
      (A) LÄNGE: 25 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 1:
   Gln Val Gln Leu Gln Gln Ser Gly Pro Glu Ala Ser Val Lys Met Ser
   1               5                   10                  15
   Cys Lys Ala Ser Gly Tyr Ile Phe Thr
               20                  25
(2) ANGABEN ZU SEQ ID NO: 2:
   (i) SEQUENZKENNZEICHEN:
      (A) LÄNGE: 25 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 2:
   Gln Val Gln Leu Gln Gln Ser Asp Ala Glu Ala Ser Val Lys Ile Ser
    1              5                   10                  15
   Cys Lys Ala Ser Gly Tyr Thr Phe Thr
               20                  25
(2) ANGABEN ZU SEQ ID NO: 3:
   (i) SEQUENZKENNZEICHEN:
      (A) LÄNGE: 25 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 3:
   Gln Val Gln Leu Gln Gln Ser Gly Ala Glu Ala Ser Val Lys Ile Ser
   1               5                   10                  15
   Cys Lys Ala Thr Gly Tyr Thr Phe Ser
               20                  25
(2) ANGABEN ZU SEQ ID NO: 4:
   (i) SEQUENZKENNZEICHEN:
      (A) LÄNGE: 25 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 4:
   Gln Val His Leu Gln Gln Ser Gly Pro Glu Pro Ser Val Lys Met Ser
    1              5                   10                  15
   Cys Lys Ala Ser Gly Tyr Thr Phe Thr
               20                  25
(2) ANGABEN ZU SEQ ID NO: 5:
   (i) SEQUENZKENNZEICHEN:
      (A) LÄNGE: 21 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 5:
   Arg Ala Thr Leu Thr Val Asp Lys Ser Ser Ser Thr Ala Tyr Met Gln
     1               5                  10                  15
   Leu Asn Ser Leu Thr
               20
(2) ANGABEN ZU SEQ ID NO: 6:
   (i) SEQUENZKENNZEICHEN:
      (A) LÄNGE: 21 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 6:
   Lys Ala Thr Leu Thr Ala Asp Lys Ser Ser Ser Thr Ala Tyr Met Gln
    1                5                  10                  15
   Leu Asn Ser Leu Thr
                20
(2) ANGABEN ZU SEQ ID NO: 7:
   (i) SEQUENZKENNZEICHEN:
      (A) LÄNGE: 21 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 7:
   Arg Ala Thr Phe Thr Ala Asp Ser Ser Ser Asn Thr Ala Phe Met Glu
    1                5                 10                   15
   Phe Gly Ser Leu Thr
               20
(2) ANGABEN ZU SEQ ID NO: 8:
   (i) SEQUENZKENNZEICHEN:
      (A) LÄNGE: 21 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 8:
   Lys Ala Ile Leu Thr Ala Asp Lys Ser Ser Ser Thr Ala Tyr Met Glu
   1               5                   10                  15
   Phe Ser Ser Leu Thr
               20
(2) ANGABEN ZU SEQ ID NO: 9:
   (i) SEQUENZKENNZEICHEN:
      (A) LÄNGE: 114 Aminosäuren
      (B) ART: Aminosäure
      (C) STRANGFORM: Einzelstrang
      (D) TOPOLOGIE: linear
   (ii) ART DES MOLEKÜLS: Peptid
      Bezeichnung: OC125
   (xi) SEQUENZBESCHREIBUNG: SEQ ID NO: 9:
   Val Gln Leu Gln Gln Ser Gly Pro Glu Leu Val Lys Pro Gly Ala Ser
   1               5                   10                  15
   Val Lys Met Ser Cys Lys Ala Ser Gly Tyr Ile Phe Thr Asp Tyr Tyr
               20                  25                  30
   Met Lys Trp Val Lys Gln Ser His Gly Arg Glu Trp Ile Gly Asp Ile
           35                  40                  45
   Asn Leu Asn Asn Gly Asp Thr Phe Tyr Asn Gln Lys Phe Lys Gly Arg
       50                  55                  60
   Ala Thr Leu Thr Val Asp Lys Ser Ser Ser Thr Ala Tyr Met Gln Leu
   65                  70                  75                  80
   Asn Ser Leu Thr Ser Glu Asp Ser Ala Val Tyr Tyr Cys Ala Arg Ser
                   85                  90                  95
   Asp Asp Tyr Gly Met Asp Tyr Trp Gly Gln Gly Thr Thr Val Thr Val
               100                 105                 110
   Ser Ser
(2) ANGABEN ZU SEQ ID NO: 10:
   (i) SEQUENZKENNZEICHEN:
      (A) LÄNGE: 114 Aminosäuren
      (B) ART: Aminosäure
      (C) STRANGFORM: Einzelstrang
      (D) TOPOLOGIE: linear
   (ii) ART DES MOLEKÜLS: Peptid
      Bezeichnung: B72.3
   (xi) SEQUENZBESCHREIBUNG: SEQ ID NO: 10:
   Gln Val Gln Leu Gln Gln Ser Asp Ala Glu Leu Val Lys Pro Gly Ala
    1               5                  10                  15
   Ser Val Lys Ile Ser Cys Lys Ala Ser Gly Tyr Thr Phe Thr Asp His
               20                  25                  30
   Ala Ile His Trp Ala Lys Gln Lys Pro Glu Gln Gly Leu Glu Trp Ile
           35                  40                  45
   Gly Tyr Ile Ser Pro Gly Asn Asp Asp Ile Lys Tyr Asn Glu Lys Phe
       50                  55                  60
   Lys Gly Lys Ala Thr Leu Thr Ala Asp Lys Ser Ser Ser Thr Ala Tyr
   65                  70                  75                  80
   Met Gln Leu Asn Ser Leu Thr Ser Glu Asp Ser Ala Val Tyr Phe Cys
                   85                  90                  95
   Lys Arg Ser Tyr Tyr Gly His Trp Gly Gln Gly Thr Thr Val Thr Val
               100                 105                 110
   Ser Ser
(2) ANGABEN ZU SEQ ID NO: 11:
   (i) SEQUENZKENNZEICHEN:
      (A) LÄNGE: 119 Aminosäuren
      (B) ART: Aminosäure
      (C) STRANGFORM: Einzelstrang
      (D) TOPOLOGIE: linear
   (ii) ART DES MOLEKÜLS: Peptid
      Bezeichnung: CD4
   (xi) SEQUENZBESCHREIBUNG: SEQ ID NO: 11:
   Gln Val His Leu Gln Gln Ser Gly Pro Glu Leu Val Lys Pro Gly Pro
    1              5                   10                  15
   Ser Val Lys Met Ser Cys Lys Ala Ser Gly Tyr Thr Phe Thr Asp Tyr
               20                  25                  30
   Val Val Ser Trp Met Gln Gln Arg Thr Gly Gln Val Leu Glu Trp Ile
           35                  40                  45
   Gly Glu Ile Tyr Pro Gly Ser Gly Ser Ala Tyr Tyr Asn Glu Lys Phe
       50                  55                  60
   Lys Gly Lys Ala Ile Leu Thr Ala Asp Lys Ser Ser Ser Thr Ala Tyr
   65                  70                  75                      80
   Met Glu Phe Ser Ser Leu Thr Ser Glu Asp Ser Ala Val Phe Phe Cys
                   85                  90                  95
   Ala Arg Arg Gly Asp Gly Ser Leu Gly Phe Ala His Trp Gly Gln Gly
               100                 105                 110
   Thr Leu Val Thr Val Ala Ala
           115
(2) ANGABEN ZU SEQ ID NO: 12:
   (i) SEQUENZKENNZEICHEN:
      (A) LÄNGE: 117 Aminosäuren
      (B) ART: Aminosäure
      (C) STRANGFORM: Einzelstrang
      (D) TOPOLOGIE: linear
   (ii) ART DES MOLEKÜLS: Peptid
      Bezeichnung: PSA-M66
   (xi) SEQUENZBESCHREIBUNG: SEQ ID NO: 12:
   Gln Val Gln Leu Gln Gln Ser Gly Ala Glu Leu Val Lys Pro Gly Ala
    1               5                  10                  15
   Ser Val Lys Ile Ser Cys Lys Ala Thr Gly Tyr Thr Phe Ser Ser Tyr
               20                  25                  30
   Trp Ile Glu Trp Met Lys Gln Arg Pro Gly His Gly Leu Glu Trp Ile
           35                  40                  45
   Gly Asp Phe Leu Pro Gly Ser Gly Ser Ser Tyr Phe Asn Glu Lys Phe
       50                  55                  60
   Lys Gly Arg Ala Thr Phe Thr Ala Asp Ser Ser Ser Asn Thr Ala Phe
   65                  70                  75                  80
   Met Glu Phe Gly Ser Leu Thr Ser Glu Asp Ser Ala Val Tyr Tyr Cys
                   85                  90                  95
   Ala Arg Arg Gly Ala Gly Arg Val Asp Tyr Trp Gly Gln Gly Thr Thr
               100                 105                 110
   Leu Thr Val Ser Ala
           115
(2) ANGABEN ZU SEQ ID NO: 13:
   (i) SEQUENZKENNZEICHEN:
      (A) LÄNGE: 123 Aminosäuren
      (B) ART: Aminosäure
      (C) STRANGFORM: Einzelstrang
      (D) TOPOLOGIE: linear
   (ii) ART DES MOLEKÜLS: Peptid
      Bezeichnung: MAK33
(2) ANGABEN ZU SEQ ID NO: 14:
   (i) SEQUENZKENNZEICHEN:
      (A) LÄNGE: 112 Aminosäuren
      (B) ART: Aminosäure
      (C) STRANGFORM: Einzelstrang
      (D) TOPOLOGIE: linear
   (ii) ART DES MOLEKÜLS: Peptid
      Bezeichnung: TSH A8
   (xi) SEQUENZBESCHREIBUNG: SEQ ID NO: 14:
   Glu Val Gln Leu Gln Glu Ser Gly Pro Asp Leu Val Lys Pro Ser Gln
   1               5                   10                  15
   Ser Leu Ser Leu Thr Cys Thr Val Thr Gly Tyr Pro Ile Thr Ser Gly
               20                  25                  30
   Tyr Thr Trp His Trp Ile Arg Gln Phe Pro Cys Leu Glu Trp Met Gly
           35                  40                  45
   Tyr Met His Tyr Asn Gly Ser Thr Asn Tyr Asn Pro Ser Leu Lys Ser
       50                  55                  60
   Arg Ile Ser Ile Thr Arg Asp Thr Ser Lys Asn Gln Phe Phe Leu Gln
   65                  70                  75                  80
   Leu Asn Ser Val Thr Thr Glu Asp Thr Ala Thr Tyr Tyr Cys Glu Phe
                   85                  90                  95
   Ser Ser Trp Asp Tyr Trp Gly Gln Gly Thr Ser Val Thr Val Ser Ala
               100                 105                 110